# EUROPEAN PATENT SPECIFICATION

(11) **EP 1 374 848 B1**
(45) Date of publication and mention of the grant of the patent: **03.09.2008**
(21) Application number: 03253622.9
(22) Date of filing: 09.06.2003
(51) Int. Cl.: A61Q 19/00, A61Q 19/10, A61K 8/02

(54) **Foaming composition comprising clay**
Schäumende Zusammensetzung enthaltend Ton
Composition moussante contentant de l'argile

(30) Priority: 10.06.2002 US 166455
(43) Date of publication of application: 02.01.2004
(73) Proprietor: NEUTROGENA CORPORATION, Los Angeles California 90045-5544 (US)
(72) Inventor: Tazberik, Erika, Huntington Beach, CA 92649 (US); Shah, Snehal, Cerritos, CA 90703 (US)
(74) Representative: Fisher, Adrian John

(56) References cited:
- EP-A- 0 595 528
- EP-A- 1 180 361
- WO-A-96/28139
- WO-A-02/102302
- GB-A- 1 255 284
- US-A- 4 388 301
- US-A- 5 846 549
- US-A- 5 968 890

## Description

### FIELD OF THE INVENTION

The present invention relates to a composition that is a cleanser and a facial mask.

### BACKGROUND OF THE INVENTION

Traditional mask compositions are non-foaming products typically used on the face for intense facial cleansing, treatment, or oil control. Examples of such masks include Neutrogena® Oil-absorbing Acne Mask and Neutrogena® Pore Refining Mask. Such masks are applied to the skin and, after a specific drying time, are rinsed off with water. The removal of such masks, however, can be difficult.

Masks are often only applied periodically, two or three times of week. Masks are also generally applied after first cleansing the skin. Thus, users are required to purchase separate products to cleanse the skin prior to application of the mask as for use on days when they do not wish to apply the mask.

Unlike traditional masks, the composition of present invention can be used both as a facial cleanser as well as a mask. The composition of the present invention, thus, can be used everyday without the need to purchase an additional cleanser. The surfactant in the product also facilitates the removal of the mask from the skin.

US 4388301 discloses face masks for use in the treatment of severe acne and having anti-seborrheic effect. These have lower levels of surfactant than in the present invention

US 5846549 concerns cosmetic compositions intended for cleaning the hair, the scalp and/or the skin.

### SUMMARY OF THE INVENTION

In one aspect, the present invention relates to a product containing a composition wherein the composition contains at least one clay, at least one foaming surfactant, and water, as set out in claim 1. In one embodiment, the product includes instructions directing the user to apply the composition to wet skin and then to remove the composition from the skin.

A user of a product containing the above composition can thus apply the composition to wet skin and then remove said composition from said skin. Alternatively the composition can be applied to dry skin, retained on the skin and then removed from the skin.

In another aspect, the present invention relates to a method of cleansing the skin including: wetting the skin; (ii) applying a composition containing at least one clay, at least one foaming surfactant, and water; and (iii) removing the composition from the skin, as set out in claim 7.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs.

What is meant by a "product" is a product in finished packaged form. In one embodiment, the package is a container such as pumps, jars, or tubes containing the composition. The product may further contain additional packaging such as a plastic or cardboard box for storing such container. In one embodiment, the product contains instructions directing the user to use the composition as a cleanser. For example, (i) to apply the composition to wet skin (e.g., to apply the composition to skin that is already wet or to apply water in conjunction with applying the composition) and (ii) then to remove the composition from the skin (e.g., to rinse the composition from the face with water). Such instructions may instruct the user to apply the composition by messaging or rubbing the composition into the skin, for example, to form lather prior to rinsing the composition.

The instructions rather or as an alternative can direct the user to apply the composition as a mask. For example, (i) to apply the composition to dry skin, (ii) to retain the composition on the skin, and (iii) to remove said composition from said skin. For example, the user may be instructed to retain the composition on the skin for a period of time to allow the composition to dry and/or for a period of time of at least one minute and less than ten minutes (e.g., such as less than about five minutes).

The instructions may also direct the user to apply the composition to the whole body or just to parts of the body such as the face, arms, or legs. Such instructions may be printed on the container, label insert, or on any additional packaging.

Examples of such instructions include, but are not limited to, "use as a cleanser," "use as a daily cleanser," "use as a mask," or "use as an intensive mask."

As used herein, "topical application" means directly laying on or spreading on outer skin using, e.g., by use of the hands or an applicator such as a wipe.

As used herein, "cosmetically-acceptable" means that the cosmetically active agents or inert ingredients which the term describes are suitable for use in contact with tissues (e.g., the skin) without undue toxicity, incompatibility, instability, irritation, allergic response, and the like, commensurate with a reasonable benefit/risk ratio.

As used herein, "safe and effective amount" means an amount of compound or composition sufficient to significantly induce a positive modification in the condition to be regulated or treated, but low enough to avoid serious side effects. The safe and effective amount of the compound or composition will vary with the particular condition being treated, the age and physical condition of the end user, the severity of the condition being treated/prevented, the duration of the treatment, the nature of concurrent therapy, the specific compound or composition employed, the particular cosmetically-acceptable topical carrier utilized, and like factors.

### Composition

The composition of the present invention includes one or more foaming surfactants. In one embodiment, the composition produces a foam volume of at least 200 ml (as defined herein in the Foaming Test), preferably, a foam volume of at least 300 ml and most preferably 400 ml.

In one embodiment, the composition has a viscosity of about 50,000 cps to about 150,000 cps measured with a Brookfield viscometer RVD VI+ equipped with a Brookfield Helipath Stand Model D at 25°C using T-C spindle at 5 rpm. The viscosity is reported as an average of 5 measurements at the given revolution going downwards and 5 measurements at the given revolution going upwards.

The composition of the present invention includes 20 to 80 percent, by weight, preferably 20 to 50 percent by weight, of water.

### Clays

The composition of the present invention includes one or more clays. In one embodiment, the clay is an aluminum silicate. Examples of aluminum silicates include, but are not limited to, bentonite, kaolin, hectorite, montmorillonite, and laponite.

The composition includes 10 to 50 percent, by weight, preferably 20 to 40 percent, by weight, of at least one clay.

### Foaming Surfactants

The composition of the present invention includes one or more foaming surfactant(s). What is meant by a foaming surfactant is a surfactant that produces a foam volume of at least 200 ml (as defined herein in the Foaming Test). Examples of foaming surfactants are found on pages 1673-89 of the International Cosmetic Ingredient Dictionary and Handbook (7^{th} Ed., The Cosmetic, Toiletry, and Fragrance Association, Washington, DC, 1997), hereinafter the ICI Handbook. In one embodiment, the foaming surfactant is an anionic, amphoteric, or nonionic surfactant. Examples of anionic foaming surfactants include, but are not limited to, sodium, magnesium, potassium, or ammonium salts of alkyl acyl taurates, alkyl sulfates, alkyl ether sulfates, olefin sulfonates, acyl isethionates, acyl sarcosinates, alkyl sulfosuccinates, and C₈-C₂₂ fatty acids. Specific examples of anionic surfactants include, but are not limited to, sodium cocoyl sarcosinate, sodium cocoyl isethionate, disodium laureth sulfosuccinate, sodium lauroyl lactylate, sodium laurate, sodium myristate, sodium palmitate, sodium methyl cocoyl taurate, and sodium lauroyl sarcosinate.

Examples of amphoteric and nonionic foaming surfactants include, but are not limited to, sodium cocoampho(di)acetate, sodium laurampho(di)acetate, sodium cocoamphopropionate, cocamidopropyl betaine, alkylpolyglucosides, and poloxamers.

The composition includes 10 to 25 percent, by weight, of said at least one foaming surfactant.

### Anti-acne Agent

In one embodiment, the composition of the present invention includes one or more antiacne agents. What is meant by an "antiacne agent" is a drug product effective is the treatment of acne. Examples of antiacne agents include, but are not limited to, azelaic acid, clindamycin, adapalene, erythromycin, sodium sulfacetamide, retinoic acid, benzoyl peroxide, sulfur, and salicylic acid.

In one embodiment, the composition includes about 0.1 to about 50 percent, by weight, of said at least one antiacne agents, e.g., about 0.5 to about 30 percent, by weight, such as about 0.5 to about 15 percent, by weight, of the at least one antiacne agent. In one embodiment, the composition further comprises a natural extract to enhance the anti-acne efficacy of the anti-acne agent. Examples of such extracts include, but are not limited to, angelica archangelica root extract, dandelion extract, turmeric extract, and melia azadirachta leaf extract.

### Viscosity Increasing Agents

In addition to the clays, the composition may further include one or more additional viscosity increasing agents. Examples of viscosity increasing agents include, but are not limited to, xanthan gum, sclerotium gum, guar gum, locust bean gum, alginates, carageenan, cellulose gum, hydroxymethyl or (ethyl or propyl)cellulose, amorphous silicon dioxide such as hydrated silica or silica, acrylic acid polymers, acrylic/acrylamide polymers, alkylene oxide polymers and esters, PVM/MA decadiene crosspolymers, and trihydroxystearin. Other examples of viscosity increasing agents can be found on pages 1693-97 of the ICI Handbook. The amount of viscosity increasing agent(s) included in the composition will depend on the desired viscosity of the composition.

### Colorants

The composition of the present invention may also include one or more colorants. The colorants may be either organic or inorganic colorants. Examples of organic colorants include, but are not limited to, various aromatic colorants including azo, indigoid, triphenylmethane, anthraquinone, and xanthine dyes which are designated as D&C and FD&C colors (e.g., blues, greens, oranges, reds, violet, and yellows). Organic colorants generally consist of insoluble metallic salts of certified color additives, referred to as the Lakes, in particular the Lakes of D&C and FD&C colors. Inorganic colorants include titanium dioxide, iron oxides, ultramarines, chromium, and chromium hydroxide colors. Other colorants are listed on pages 1628-30 of the ICI Handbook. The amount of colorant(s) will depend on the desired color of the composition.

### Cooling Agents

In one embodiment, the composition includes one or more cooling agents. What is meant by a "cooling agent" is an ingredient which when topically applied on skin creates a sensation of coolness and freshness . Examples of cooling agents include, but are not limited to, menthol, methyl lactate, menthone glycerin acetal, camphor, peppermint leaf extract, and balm mint leaf extract.

In one embodiment, the composition includes about 0.01 to about 2 percent, by weight, of said at least one cooling agent, e.g., about 0.05 to about 1 percent, by weight, such as about 0.05 to about 0.5 percent, by weight, of the at least one cooling agent.

### Skin Conditioning Agents

In one embodiment, the composition includes one or more skin conditioning agents. Examples of skin conditioning agents include, but are not limited to, emollients and humectants such as propylene glycols, glycerin, and polyethylene glycols such as Trideceth-9 and PEG-5 Ethylhexanoate. Other skin conditioning agents are listed on pages 1656-70 of the ICI Handbook.

In one embodiment, the composition includes about 0.01 to about 30 percent, by weight, of said at least one skin conditioning agent, e.g., about 0.1 to about 20 percent, by weight (such as about 0.5 to about 15 percent, by weight) of the at least one skin conditioning agent.

### Other Ingredients

The composition may further comprise one or more chelating agents such as disodium EDTA, pH adjusters such as citric acid and sodium citrate, vitamins such as vitamins A, Bs, C, and E, fragrances, and preservatives such as parabens and phenoxyethanol. Examples of such are disclosed on pages 1626, 1639-40, and 1653-55 of the ICI Handbook.

### Foaming Test

The follow test is used to determine the foam volume of the test composition (e.g., the foaming surfactant or the composition). One gram of the test composition is added into a beaker with ninety-nine grams of deionized water. The mixture is then mixed with a magnetic stir bar until completely homogeneous. The resulting solution is then transferred into a 500 ml graduated cylinder with a glass stopper. The cylinder is manually and vigorously shaken for 1 minute and placed down on to a bench top for one minute. The volume of the foam layer is then measured in graduated cylinder (foam volume).

### Examples

The following is a description of the manufacture of compositions of the present invention, when the clay, foaming surfactants and water are present in amounts as set out in claim 1 and 7. Other compositions of the invention can be prepared in an analogous manner by a person of ordinary skill in the art.

### Example 1:

The compositions of Table 1 can be manufactured in the following manner:

**Table 1**

| Item # | CHEMICAL NAME (INCI) | % (W/W) | Supplier |
|---|---|---|---|
| 1 | Water | q.s. 100 | |
| 2 | Xanthan Gum | 0.1 - 5 | Keltrol CG Calgon Corporation Pittsburgh, PA USA |
| 3 | Glycerin | 0.1 - 30 | Glycerin 00.7% Cognis Cincinnati, OH USA |
| 4 | Disodium EDTA | 0.01 - 1 | Hamp-ene Na2 Hampshire Chemical Corp. Nashua, NH USA |
| 5 | Kaolin | 0.1 - 25 | Kaolin Kaopolite, Inc. Union, NJ USA |
| 6 | Titanium Dioxide | 0.1 - 5 | Titanium Dioxide Sun Chemical Corporation Cincinnati, OH USA |
| 7 | Bentonite | 0.1 - 25 | Polargel HV BC Whittaker, Clark & Daniels, Inc. South Plainfield, NJ USA |
| 8 | Citric Acid | 0.01-2 | Citric Acid Anhydrous Tate & Lyle Decatur, IL USA |
| 9 | Sodium Citrate | 0.01-1 | Sodium Citrate FCC Tate & Lyle |
| 10 | Benzoyl Peroxide | 0.1-15 | Cadet BPO-78 USP Akzo Nobel Chicago, IL USA |
| 11 | Sodium Methyl Cocoyl Taurate | 0.1- 50 | Tauranol WS Finetex, Inc. Elmwood Park, NJ |
| 12 | Sodium Lauroyl Sarcosinate | 0.1-50 | Hamposyl L-30 Hampshire Chemical Corp. Nashua NH 03060 |
| 13 | Sodium Cocoamphoaceta te | 0.1-50 | Mackam HPC-32 McIntyre Group Ltd University Park IL 60466 |
| 14 | Trideeth-9 (and) PEG-5 Ethylhexanoate | 0.01-5 | NEO-PLC W/S Dragoco Totowa, NJ USA |
| 15 | Menthol | 0.01-2 | Menthol Crystals Technology Flavors and Fragrances Amityville, NY USA |
| 16 | Fragrance | 0.01-2 | Quest International Fragrances USA, Inc. Mount Olive, NJ |

Items 1, 2, and 3 are added into a batch tank and heated and mixed at 55-60°C for a minimum of 30 minutes. When the mixture is free of lumps, item 4 is added and homogeneously mixed. The batch is then re-circulated, and the temperature is maintained at 45-55°C. Items 5, 6, and 7 are then slowly added to the mixture, and the mixture is mixed at 45-55°C until the mixture is smooth. Items 8 and 9 are then added to the mixture. The batch is then cooled to 28-30°C. When the temperature of the mixture has cooled to 30°C, item 10 is added and mixed until uniform. Item 10 ("BPO") is added as a phase, which is prepared by milling BPO with a portion of the water and glycerin until the particle size of the BPO has been reduced to less than 10µm. Then, item 11, 12, and/or 13 is added during mixing. When the mixture is uniform, item 14, 15, and 16 are premixed and then added to the mixture.

### Example 2:

The following three compositions (Formulations A, B, and C) of Table 2 were manufactured in the manner set forth in Example 1.

**Table 2**

| Item # | CHEMICAL NAME (INCI) | Form. A | Form. B | Form. C |
|---|---|---|---|---|
| 1 | Water | 45.08 | 45.08 | 45.08 |
| 2 | Xanthan Gum | 0.6 | 0.6 | 0.6 |
| 3 | Glycerin | 12.12 | 12.12 | 12.12 |
| 4 | Disodium EDTA | 0.2 | 0.2 | 0.2 |
| 5 | Kaolin | 11 | 11 | 11 |
| 6 | Titanium Dioxide | 3 | 3 | 3 |
| 7 | Bentonite | 9 | 9 | 9 |
| 8 | Citric Acid | 0.4 | 0.4 | 0.4 |
| 9 | Sodium Citrate | 0.3 | 0.3 | 0.3 |
| 10 | Benzoyl Peroxide | 4.5 | 4.5 | 4.5 |
| 11 | Sodium Methyl Cocoyl Taurate | 13 | 0 | 0 |
| 12 | Sodium Lauroyl Sarcosinate | 0 | 13 | 0 |
| 13 | Sodium Cocoamphoacetate | 0 | 0 | 13 |
| 14 | Trideeth-9 (and) PEG-5 Ethylhexanoate | 0.5 | 0.5 | 0.5 |
| 15 | Menthol | 0.1 | 0.1 | 0.1 |
| 16 | Fragrance | 0.2 | 0.2 | 0.2 |

### Example 3:

Formulations A, B, and C of Example 2 were subjected to the Foaming Test described herein. Formulation A had a foam volume of 450 ml, formulation B had a foam volume of 345 ml, and formulation C had a foam volume of 475 ml.

## Claims

1. A composition comprising 10 to 50 percent, by weight, of at least one clay, 10 to 25 percent, by weight, of at least one foaming surfactant, and 20 to 80 percent, by weight, of water, wherein said composition is a cleanser and a facial mask.

2. The composition of claim 1, wherein at least one of said foaming surfactants is an anionic surfactant.

3. The composition of claim 1, wherein said composition has a foam volume of at least 200 ml.

4. The composition of claim 1 comprising an anti-acne agent.

5. The composition of claim 4, wherein said anti-acne agent is benzoyl peroxide, salicylic acid, or a mixture thereof.

6. The composition of claim 1, wherein said at least one clay is kaolin, bentonite, or a mixture thereof.

7. A method of cleansing the skin, said method comprising:
(i) wetting said skin;
(ii) applying a composition comprising 10 to 50 percent, by weight, of at least one clay, 10 to 25 percent, by weight, of at least one foaming surfactant, and 20 to 80 percent, by weight, of water to the skin;
(iii) retaining said composition on the skin for at least one minute and less than ten minutes; and
(iv) removing said composition from said skin.

8. The method of claim 7, wherein at least one of said foaming surfactants is an anionic surfactant.

9. The method of claim 7, wherein said composition has a foam volume of at least 200 ml.

10. The method of claim 7, wherein said composition comprises an anti-acne agent.

11. The method of claim 7, wherein said at least one clay is kaolin, bentonite, or a mixture thereof.

## Patentansprüche

1. Zusammensetzung, die 10 bis 50 Gewichtsprozent von wenigstens einem Ton, 10 bis 25 Gewichtsprozent von wenigstens einem schäumenden Tensid und 20 bis 80 Gewichtsprozent Wasser umfasst, wobei die Zusammensetzung ein Reinigungsmittel und eine Gesichtsmaske ist.

2. Zusammensetzung nach Anspruch 1, wobei wenigstens eines der schäumenden Tenside ein anionisches Tensid ist.

3. Zusammensetzung nach Anspruch 1, wobei die Zusammensetzung ein Schaumvolumen von wenigstens 200 ml besitzt.

4. Zusammensetzung nach Anspruch 1, die ein Antiaknemittel umfasst.

5. Zusammensetzung nach Anspruch 4, wobei das Antiaknemittel Benzoylperoxid, Salicylsäure oder eine Mischung davon ist.

6. Zusammensetzung nach Anspruch 1, wobei der wenigstens eine Ton Kaolin, Bentonit oder eine Mischung davon ist.

7. Verfahren zur Reinigung der Haut, wobei das Verfahren umfasst:
(i) Benetzen der Haut;
(ii) Aufbringen einer Zusammensetzung, die 10 bis 50 Gewichtsprozent von wenigstens einem Ton, 10 bis 25 Gewichtsprozent von wenigstens einem schäumenden Tensid und 20 bis 80 Gewichtsprozent Wasser umfasst, auf die Haut;
(iii) Halten der Zusammensetzung auf der Haut für wenigstens eine Minute und weniger als zehn Minuten; und
(iv) Entfernen der Zusammensetzung von der Haut.

8. Verfahren nach Anspruch 7, wobei wenigstens eines der schäumenden Tenside ein anionisches Tensid ist.

9. Verfahren nach Anspruch 7, wobei die Zusammensetzung ein Schaumvolumen von wenigstens 200 ml besitzt.

10. Verfahren nach Anspruch 7, wobei die Zusammensetzung ein Antiaknemittel umfasst.

11. Verfahren nach Anspruch 7, wobei der wenigstens eine Ton Kaolin, Bentonit oder eine Mischung davon ist.

## Revendications

1. Composition comprenant 10 à 50 pour cent, en poids, d'au moins une argile, 10 à 25 pour cent, en poids, d'au moins un tensioactif moussant, et 20 à 80 pour cent, en poids, d'eau, dans laquelle ladite composition est un nettoyant et un masque facial.

2. Composition selon la revendication 1, dans laquelle au moins un desdits tensioactifs moussants est un tensioactif anionique.

3. Composition selon la revendication 1, dans laquelle ladite composition a un volume de mousse d'au moins 200 mL.

4. Composition selon la revendication 1, comprenant un agent anti-acnéique.

5. Composition selon la revendication 4, dans laquelle ledit agent anti-acnéique est le peroxyde de benzoyle, l'acide salicylique ou leur mélange.

6. Composition selon la revendication 1, dans laquelle ladite au moins une argile est le kaolin, la bentonite ou leur mélange.

7. Méthode de nettoyage de la peau, ladite méthode comprenant :
(i) le mouillage de ladite peau ;
(ii) l'application d'une composition comprenant 10 à 50 pour cent, en poids, d'au moins une argile, 10 à 25 pour cent, en poids, d'au moins un tensioactif moussant, et 20 à 80 pour cent, en poids, d'eau sur la peau ;
(iii) le maintien de ladite composition sur la peau pendant au moins une minute et moins de dix minutes ; et
(iv) le retrait de ladite composition de ladite peau.

8. Méthode selon la revendication 7, dans laquelle au moins un desdits tensioactifs moussants est un tensioactif anionique.

9. Méthode selon la revendication 7, dans laquelle ladite composition a un volume de mousse d'au moins 200 mL.

10. Méthode selon la revendication 7, dans laquelle ladite composition comprend un agent anti-acnéique.

11. Méthode selon la revendication 7, dans laquelle ladite au moins une argile est le kaolin, la bentonite ou leur mélange.
